(19) Europäisches Patentamt
European Patent Office
Office européen des brevets

(11) Publication number: **0 014 440**
**A1**

(12) # EUROPEAN PATENT APPLICATION

(21) Application number: **80100489.6**

(22) Date of filing: **31.01.80**

(51) Int. Cl.³: **C 07 D 333/22**
C 07 D 409/04

(30) Priority: **02.02.79 JP 10484/79**

(43) Date of publication of application:
**20.08.80 Bulletin 80/17**

(84) Designated Contracting States:
**AT BE CH DE FR GB IT NL SE**

(71) Applicant: **SAGAMI CHEMICAL RESEARCH CENTER**
**4-5, Marunouchi 1-chome**
**Chiyoda-ku, Tokyo, 100(JP)**

(72) Inventor: **Kondo, Kiyosi**
**16-4, Chuorinkan 5-chome**
**Yamato-shi Kanagawa-ken(JP)**

(72) Inventor: **Matsui, Kiyohide**
**9-2, Minamidai 1-chome**
**Sagamihara-shi Kanagawa-ken(JP)**

(72) Inventor: **Sugimoto, Kikuo**
**1780, Sanogawa, Fujino-machi**
**Tsukui-gun Kanagawa-ken(JP)**

(74) Representative: **Hansen, Bernd, Dr.rer.nat. et al,**
**Hoffmann . Eitle & Partner Patentanwälte**
**Arabellastrasse 4**
**D-8000 München 81(DE)**

(54) **Ketal derivatives and the use thereof as intermediate products.**

(57) Ketal derivative having the formula:

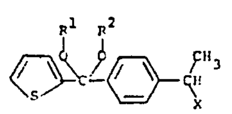

in which $R^1$ and $R^2$ individually represent alkyl groups, or $R^1$ and $R^2$ may together form an alkylene group, and X is a hydrogen atom, a halogen atom or a cyano group, and the use thereof as intermediate products for producing Suprofen.

EP 0 014 440 A1

Ketal derivatives and the use thereof as intermediate products.

This invention relates to a ketal derivative and the use thereof as intermediate products. Particularly the invention relates to a ketal derivative that can be employed as an intermediate product for preparing Suprofen (a registered trademark) which shows prominent analgesic and anti-inflammatory effects.

Heretofore known processes for preparing Suprofen are as follows:
(1) a process in which p-fluorobenzoyl chloride is reacted sequently with thiophene and methyl malonate (See German Offenlegungsschrift P 23 53 357);
(2) a process in which fluorobenzene is reacted with 2-thenoyl chloride and the resulting product then is reacted with methyl malonate (See Chemical Abstracts 84, 43737x);
(3) a process in which a phenylacetylene derivative is treated with thallium nitrate (See Japanese Patent Provisional Publication (JPPP) No. 52-36642);
(4) a process in which a diester is, in the first stage, alkylated and then subjected to hydrolysis and decarboxylation (See JPPP No. 49-93346);
(5) a process in which an acetophenone derivative is subjected to Willgerodt reaction in the presence of rhodanine (See JPPP No. 49-93346);
(6) a process in which an α-haloethylbenzene derivative is subjected to cyanation followed by hydrolysis (See JPPP No. 49-93346 and P.G.H. Van Daele et al., Arzneim-Forsch, 25, 1495 (1975)); and others.

These process have certain drawbacks such as rather long reaction route, the use of starting materials not so readily available, the involvement of reaction stages giving low yields, and so forth. Therefore, these processes do not appear to be ones industrially applicable.

In the processes, the process (6) is considered to be most industrially advantageous and the process (6) is illustrated as follows:

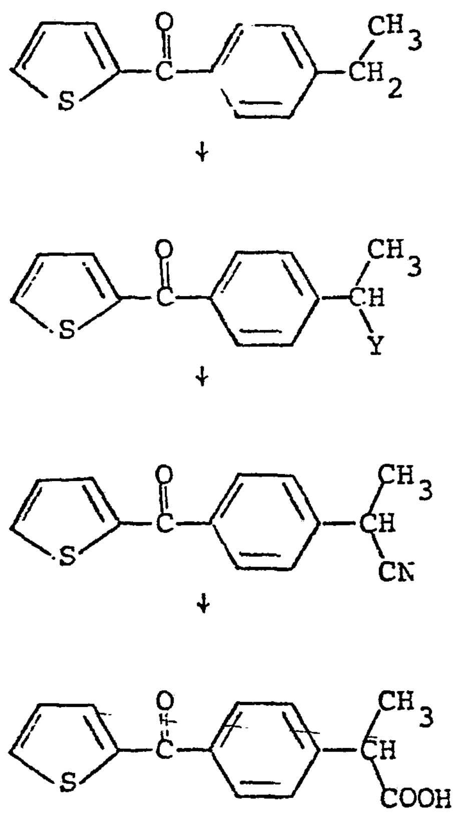

(1)

(2)

(3)

(4) .......Suprofen

in which Y is a halogen atom.

In this synthetic route, the reaction of the compound (2), i.e., (1-halogenoethyl)phenyl thienyl ketone, to yield the compound (3), i.e., (1-cyanoethyl)phenyl thienyl ketone, also gives a substantial amount of a by-product having the formula (2'):

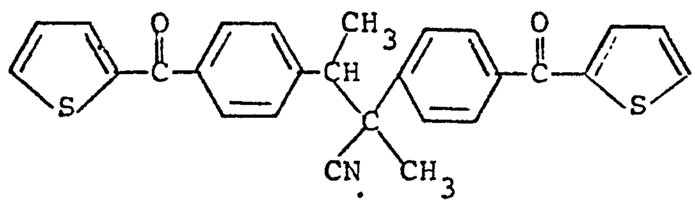

(2')

and this means a decreased yield of the compound (2) and also means a decreased yield of the compound (4), i.e., Suprofen, which is naturally disadvantageous.

As a result of earnest studies on intermediates for obtaining Suprofen in good yield which then obviates the drawbacks mentioned above, the present inventors have achieved this invention upon finding that a ketal derivative in which the carbonyl group in the para-position is converted to a ketal can produce Suprofen in a high yield.

Accordingly, the primary object of this invention is to provide a novel ketal derivative of value as an intermediate for preparing Suprofen and such use. Other objects will be apparent from the contents of this specification.

The ketal derivative of this invention has the formula [I]:

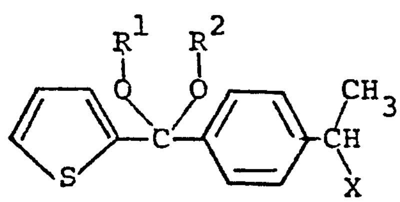

[I]

in which $R^1$ and $R^2$ individually represent alkyl groups, or $R^1$ and $R^2$ may together form an alkylene group, and X is a hydrogen atom, a halogen atom or a cyano group.

In the formula [I], each of $R^1$ and $R^2$ is the same or different, and preferably represents a lower alkyl group of from 1 to 4 carbon atoms, such as methyl, ethyl,

n-propyl, isopropyl and n-butyl. Preferred examples of the alkylene group include substituted or unsubstituted dimethylene or trimethylene groups of from 2 to 6 carbon atoms such as ethylene, 1-methylethylene, propylene, 2-methylpropylene and 2,2-dimethylpropylene. The halogen included within the symbol X is preferred to be chlorine or bromine.

Examples of the compound [I] of this invention include: (4-ethylphenyl)(2-thienyl)dimethoxymethane, (4-ethylphenyl)-(2-thienyl)diethoxymethane, (4-ethylphenyl)(2-thienyl)di-n-butylmethane, 2-[(4-ethylphenyl)(2-thienyl)]-1,3-dioxolane, 2-[(4-ethylphenyl)(2-thienyl)]-1,3-dioxane, 2-[(4-ethylphenyl)(2-thienyl)]-5,5-dimethyl-1,3-dioxane, [4-(1-chloroethyl)phenyl](2-thienyl)dimethoxymethane, [4-(1-bromoethyl)phenyl](2-thienyl)dimethoxymethane, [4-(1-chloroethyl)phenyl](2-thienyl)diethoxymethane, [4-(1-bromoethyl)phenyl](2-thienyl)diethoxymethane, [4-(1-chloroethyl)phenyl](2-thienyl)-1,3-dioxolane, [4-(1-bromoethyl)phenyl](2-thienyl)-1,3-dioxolane, [4-(1-cyanoethyl)phenyl](2-thienyl)dimethoxymethane, [4-(1-cyanoethyl)phenyl](2-thienyl)diethoxymethane, and 2-{[4-(1-cyanoethyl)phenyl](2-thienyl)}-1,3-dioxolane.

A compound of the formula [I] according to this invention can be synthesized, for example, by the following steps.

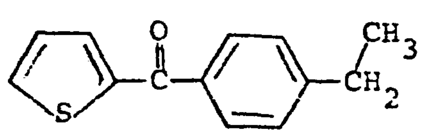

[II]

First step Ketalation

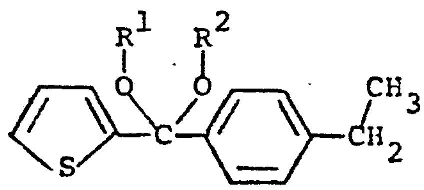

[I - 1]

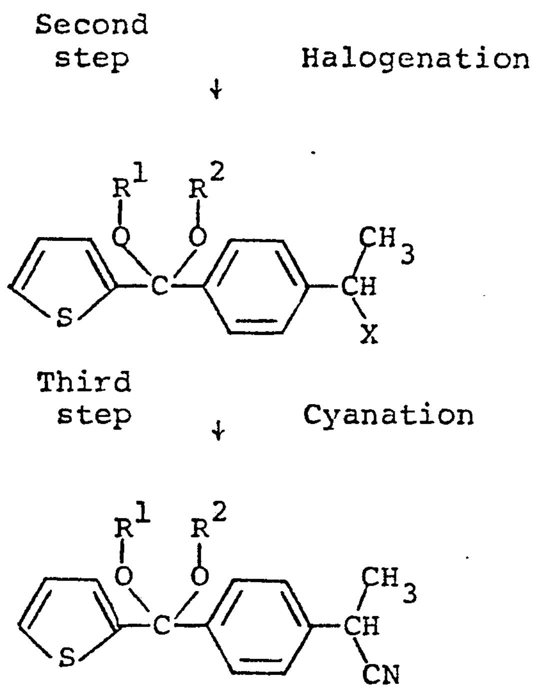


[I - 2]

[I - 3].

In the above formulae [I - 1], [I - 2] and [I - 3], $R^1$, $R^2$ and X have the same meanings as defined hereinbefore.

The first step is directed to the synthesis of the compound [I - 1], i.e., (4-ethylphenyl)(2-thienyl)-dialkoxymethane which comprises the reaction of the compound [II], i.e., ethylphenyl thienyl ketone, with an alcohol or ortho-ester. The compound [II] of the starting compound can be readily prepared by the reaction of ethylbenzene with thenoyl chloride or the reaction of p-ethylbenzoyl chloride with thiophene under the Friedel-Crafts reaction conditions. (See N. P. BunHoi et al., Bull. Soc. Chim. Fr., 447 (1959)). Examples of the alcohol employed include lower alcohols of from 1 to 4 carbon atoms, such as methanol, ethanol, n-propanol, isopropanol and butanol. Each of the alcohols can be employed alone or in combination. Examples of the ortho-ester include methyl orthoformate, ethyl orthoformate, ethyl orthoacetate and ethyl orthosilicate.

The reaction of the first step is preferably carried out

in the presence of an acidic substance so as to accelerate its reaction rate. Examples of the acidic substance include organic acids such as acetic acid, and p-toluenesulfonic acid; mineral acids such as hydrochloric acid and sulfuric acid; Lewis acids such as iron chloride, zinc chloride and titanium tetrachloride; ammonium salts such as ammonium sulfate and ammonium chloride; and acidic resins such as Amberlist*- 15 (trade name, Rohm & Haas Company). The acidic substance can be employed in a catalytic or equivalent amount as compared with the amount of the starting compound (compound [I]). For carrying out this reaction, a solvent can be employed. Examples of the solvent, that do not participate in the reaction, include hydrocarbon-type solvents such as hexane, benzene, toluene and xylene; ether-type solvents such as ether and dioxane; and halogenated hydrocarbon-type solvents such as carbon tetrachloride, in addition to the aforementioned alcohols involved in the reaction. The reaction can be carried out under cooling in ice or a temperature up to 200 °C. Room temperature or the refluxing temperature of the solvent employed is preferably adopted for facilitating the procedures.

The second step is directed to the synthesis of the compound [I-2], i.e., [4-(1-halogenoethyl)phenyl](2-thienyl)-dialkoxymethane which comprises the reaction of the compound [I - 1] obtained in the first step, i.e., (4-ethylphenyl)(2-thienyl)dialkoxymethane, with a halogenating agent. Examples of the halogenating agent include halogenated imides such as N-bromosuccinimide and N-chlorosuccinimide; chlorine; bromine; t-butyl hypobromide; trichloromethanesulfonyl chloride; and trichlorobromomethane. The reaction of the second step is preferably carried out under conditions producing radicals so that the reaction can be accelerated and that the yield of the product can be increased. The conditions producing

* a registered trademark

radicals can be readily attained, for example, by causing a radical producing agent present in the reaction mixture or placing the reaction mixture under the radiation of light. Examples of the radical producing agent include organic peroxides such as benzoyl peroxide, diacetyl peroxide and di-t-butyl peroxide; azo compounds such as azobisisobutyronitrile; and transition metal compounds such as iron chloride, copper oxide, palladium complexes and rhodium complexes.

The reaction is preferably carried out in the presence of a solvent. Examples of the solvent that can be employed include halogenated hydrocarbons such as carbon tetrachloride and chloroform; aromatic hydrocarbons such as benzene; and other hydrocarbons such as hexane and cyclohexane. The solvent ought not to participate in the reaction.

The reaction temperature may vary depending upon the reaction conditions actually employed, but generally is between room temperature and 150 °C. The reaction temperature preferably ranges from room temperature to temperatures around the reflux temperature of the solvent employed, for facilitating the procedures.

The third step is directed to the synthesis of the compound [I - 3], i.e., [4-(1-cyanoethyl)phenyl](2-thienyl)-dialkoxymethane which comprises the reaction of the compound [I - 2] obtained in the second step, i.e., [4-(1-halogenoethyl)phenyl](2-thienyl)dialkoxymethane, with a metallic cyanide. Examples of the metallic cyanide that can be employed include sodium cyanide, potassium cyanide and copper cyanide.

The reaction of this step is preferably carried out in the presence of a solvent. Examples of the solvent that

can be employed include aprotic polar solvents such as dimethylsulfoxide, dimethylformamide and hexamethylphosphoramide; alcohols such as methanol and ethanol; and aromatic hydrocarbons such as benzene and toluene. An aprotic polar solvent is preferably employed for rendering the reaction smooth.

The compound [III] obtained in this step, i.e., [4-(1-cyanoethyl)phenyl](2-thienyl)dialkoxymethane, can be hydrolyzed under an acidic condition to produce the Suprofen in a good yield. In this reaction for preparing Suprofen, the acidic condition can be set by incorporating an acidic substance in the reaction mixture. Examples of the acidic substance include mineral acids such as hydrochloric acid, sulfuric acid and phosphoric acid; and organic acids such as formic acid and acetic acid. A mineral acid is preferably employed, because the mineral acid is economical and can render the reaction smooth. The organic acid can also serve as a solvent. The amount of the acidic substance employed is not limited and may vary depending upon the reaction conditions. The amount of the acidic substance accordingly is between a catalytic amount and a very excessive one as compared with the amount of the starting cyano compound, i.e., the compound [I - 3].

In carrying out the hydrolysis reaction, water is employed in an amount of not less than three times the molar equivalents of the starting compound [I - 3]. If necessary, a water-miscible solvent such as an organic solvent stated hereinbefore or an ether, e.g., dioxane or dimethoxyethane can be employed in conjunction with the water. The reaction can be carried out at a temperature between room temperature and 150 °C., and a temperature around 100 °C. or the reflux temperature of the solvent employed is preferably used.

The present invention will be further illustrated by the Comparative Example, Synthesis Examples, Examples embodying the invention and Reference Examples, all of which are in no way limiting the present invention.

Comparative Example 1.

To 3.25 g. of dry dimethylsulfoxide was added 0.289 g. of sodium cyanide, and the mixture was heated to 60 °C. to dissolve the latter in the former. To the resulting solution was added 0.59 g. of (1-bromoethyl)phenyl thienyl ketone (prepared in the manner as described in Japanese Patent Provisional Publication No. 49-93346), and the mixture was stirred at 60 °C. for 4.5 hours. The reaction mixture was then treated in the manner as described in JPPP No. 49-93346, Example 17. The crude product thus obtained was purified over silica gel column to give 0.12 g. of (1-cyanoethyl)phenyl thienyl ketone. Yield 25%.

Synthesis Example 1.

To a mixture of 20 mℓ. of ethylbenzene and 7.33 g. of 2-thenoyl chloride was added portion-wise 10 g. of powdery anhydrous aluminum chloride at room temperature. After the addition was complete, the reaction mixture was stirred at room temperature for 2 hours and poured into a mixture of ice and water. The mixture was then extracted with ether, and the separated ether phase was washed successively with water, dilute hydrochloric acid, aqueous sodium hydrogen carbonate and aqueous sodium chloride, and dried. The dried solution was filtered and the filtrate was concentrated to leave a crude residue, which in turn was distilled under reduced pressure to give 8.97 g. of 4-(2-thenoyl)ethylbenzene, b.p. 144 - 145 °C/0.3 mmHg. Yield 83%.

NMR spectrum (in carbon tetrachloride): δ 7.8 - 7.4 (m, 4H), 7.35 - 6.9 (m, 3H), 2.65 (q, 2H), 1.17 (t, 3H).

Example 1.

In anhydrous ethanol were dissolved 2.4 g. of 4-(2-thenoyl)ethylbenzene (prepared in the manner as described in Synthesis Example 1) and 5 mℓ. of ethyl orthoformate, and three drops of concentrated sulfuric acid were added. The resulting solution was then heated to reflux for 10 hours under stirring. The mixture was cooled and neutralized with powdery sodium carbonate, and then concentrated under reduced pressure. The residue was poured into aqueous sodium hydrogen carbonate, and the mixture was subjected to extraction with ether. The ether phase was separated, washed with aqueous sodium chloride and dried over anhydrous sodium carbonate. The dried extract was filtered, and the filtrate was concentrated and distilled under reduced pressure to give 2.935 g. of (4-ethylphenyl)(2-thienyl)diethoxymethane, b.p. 117 °C/0.2 mmHg. Yield 91%.

NMR spectrum (in carbon tetrachloride): δ 7.5 - 6.9 (m, 5H), 6.9 - 6.7 (m, 2H), 3.35 (q, 4H), 2.62 (q, 2H), 1.22 (t, 3H), 1.20 (t, 6H).

Example 2.

To a mixture of 1.45 g. of (4-ethylphenyl)(2-thienyl)-diethoxymethane, 0.98 g. of N-bromosuccinimide and 10 mℓ. of carbon tetrachloride was added 30 mg. of benzoyl peroxide. The resulting mixture was heated under the argon atmosphere to 80 °C., and stirred for one hour at the same temperature. The mixture was cooled and, after addition of powdery sodium carbonate, was stirred for a while and filtered to remove insolubles. The filtrate

was concentrated under reduced pressure to give 1.845 g. of crude [4-(1-bromoethyl)phenyl](2-thienyl)diethoxymethane.

NMR spectrum (in carbon tetrachloride): δ 7.44 (d, 1H), 7.27 (d, 1H), 7.07 (t, 1H), 6.78 (d, 2H), 5.05 (q, 1H), 3.32 (q, 4H), 1.97 (d, 3H), 1.18 (t, 6H).

Example 3.

To 7.5 mℓ. of dry dimethylsulfoxide was added 725 mg. of sodium cyanide, and the mixture was heated to 60 °C. to bring it to solution. To the resulting solution was added dropwise but quickly a solution of 1.785 g. of the crude product obtained in Example 2, i.e., [4-(1-bromoethyl)phenyl](2-thienyl)diethoxymethane, dissolved in 1 mℓ. of dry dimethylsulfoxide, at a temperature of 60 °C. The mixture was further heated under stirring for about one hour, cooled and poured into water. The resulting aqueous mixture was extracted three times with ether, and the ether phase was separated. This phase was washed with water and aqueous sodium chloride and dried over anhydrous magnesium sulfate. The dried phase was filtered, and the filtrate was concentrated. The resulting crude product was purified over silica gel column to give 960 mg. of [4-(1-cyanoethyl)phenyl](2-thienyl)diethoxyethane. Yield (overall yield through both the bromination and the cyanation) 63%.

NMR spectrum (in carbon tetrachloride): δ 7.50 (d, 2H), 7.19 (d, 2H), 7.06 (t, 1H), 6.79 (d, 2H), 3.71 (q, 1H), 3.30 (q, 4H), 1.53 (d, 3H), 1.37 (t, 6H).

Example 4.

In 10 mℓ. of dry benzene was dissolved 1.1 g. of 4-(2-

thenoyl)ethylbenzene obtained in Synthesis Example 1, and to this solution were added 2 mℓ. of ethylene glycol and 10 mg. of p-toluenesulfonic acid. The resulting mixture was then heated under stirring. The water produced in the course of the reaction was removed from the reaction mixture by means of the Dean-Stark reaction apparatus. The reaction was continued overnight, and the reaction mixture was poured into aqueous sodium hydrogen carbonate and subjected to extraction with ether. The ether phase was separated, washed with water and aqueous sodium chloride, and dried. The dried phase was filtered, and the filtrate was concentrated to give 1.29 g. of 2-[(4-ethylphenyl)(2-thienyl)]-1,3-dioxolane, b.p. 173 - 174 °C/1.2 mmHg.

Example 5.

To a mixture of 1.23 g. of 2-[(4-ethylphenyl)(2-thienyl)]-1,3-dioxolane obtained in Example 4, 925 mg. of N-bromosuccinimide and 10 mℓ. of carbon tetrachloride was added 20 mg. of benzoyl peroxide. The resulting mixture was heated to reflux under the argon atmosphere for about 3 hours. The mixture was cooled and, after addition of about 5 mℓ. of dry hexane, filtered to remove insolubles. The filtrate was concentrated under reduced pressure to give 1.55 g. of 2-{[4-(1-bromoethyl)phenyl](2-thienyl)}-1,3-dioxolane.

NMR spectrum (in carbon tetrachloride): $\delta$ 7.43 (d, 2H), 7.32 (d, 2H), 7.10 (m, 1H), 6.9 - 6.7 (m, 2H), 5.03 (q. 1H), 4.15 - 3.7 (m, 4H), 1.94 (d, 3H).

Example 6.

In 2 mℓ. of dry dimethylsulfoxide was dissolved 1.50 g. of 2-{[4-(1-bromoethyl)phenyl](2-thienyl)}-1,3-dioxolane obtained in Example 5. The resulting solution was added

dropwise but quickly to a solution of 0.5 g. of sodium cyanide dissolved in 2 mℓ. of dry dimethylsulfoxide heated to 90 °C. in advance of the addition. The mixture was further heated under stirring for about 40 minutes and then treated in the manner as stated in Example 3. The crude product thus obtained was purified over silica gel column to give 0.74 g. of 2-{[4-(1-cyanoethyl)phenyl](2-thienyl)}-1,3-dioxolane. Yield (overall yield through both the bromination and the cyanation) 57%.

NMR spectrum (in carbon tetrachloride): δ 7.52 (d, 2H), 7.20 (d, 2H), 7.15 (m, 1H), 6.9 - 6.65 (m, 2H), 4.15 - 3.8 (m, 4H), 3.73 (q, 1H), 1.47 (d, 3H).

Reference Example 1.

In 1 mℓ. of dioxane was dissolved 317 mg. of [4-(1-cyanoethyl)phenyl](2-thienyl)diethoxymethane obtained in Example 3. To the resulting solution was added 4 mℓ. of 50% sulfuric acid, and the mixture was then heated to reflux for 2 hours. After addition of 4 mℓ. of water, the mixture was further heated to reflux for 2 hours under stirring. The mixture was then cooled and, after addition of a great amount of water, was subjected to extraction with ethyl acetate. The organic phase was separated, washed with aqueous sodium chloride and dried. The dried phase was filtered, and the filtrate was concentrated to give 245 mg. of a crude product. The crude product was then recrystallized from acetonitrile to give 220 mg. of α-methyl-4-(2-thenoyl)phenylacetic acid (Suprofen). Yield 85%. M.p. 120 - 121 °C.

NMR spectrum (in deuterochloroform): δ 11.5 (s, 1H), 7.77 (d, 2H), 7.62 (d, 1H), 7.57 (d, 1H), 7.43 (d, 2H), 7.07 (t, 1H), 3.79 (q, 1H), 1.53 (d, 3H).

Reference Example 2.

The procedures stated in Reference Example 1 were repeated using 285 mg. of 2-{[4-(1-cyanoethyl)phenyl](2-thienyl)}-1,3-dioxolane obtained in Exampl 6 to give 205 mg. of α-methyl-4-(2-thenoyl)phenylacetic acid (Suprofen). Yield 79%.

Claims:

1. A ketal derivative having the formula [I]:

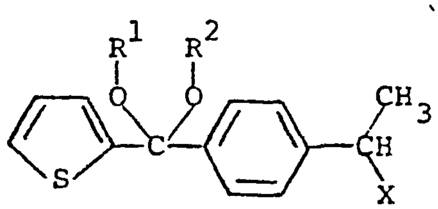

[I]

in which $R^1$ and $R^2$ individually represent alkyl groups, or $R^1$ and $R^2$ may together form an alkylene group, and X is a hydrogen atom, a halogen atom or a cyano group.

2. A ketal derivative as claimed in Claim 1 wherein the alkyl group is a lower alkyl group of from 1 to 4 carbon atoms and the alkylene group is a substituted or unsubstituted dimethylene or trimethylene group of from 2 to 6 carbon atoms.

3. A ketal derivative as claimed in Claim 1 wherein the halogen atom is chlorine or bromine.

4. The use of a ketal derivative according to any of claims 1-3 as an intermediate product for preparing Suprofen.

0014440

European Patent Office

# EUROPEAN SEARCH REPORT

Application number
EP 80 10 0489

## DOCUMENTS CONSIDERED TO BE RELEVANT

| Category | Citation of document with indication, where appropriate, of relevant passages | Relevant to claim |
|---|---|---|
| A | GB - A - 1 395 666 (ROUSSEL-UCLAF)<br>* Page 1, lines 15-30, 46-70; page 2, lines 119-123; page 3, lines 1-35 *<br>-- | 1 |
| P,A | GB - A - 1 539 897 (ORCHIMED)<br>* Claims 1,5 *<br>---- | 1 |

CLASSIFICATION OF THE APPLICATION (Int. Cl.)

C 07 D 333/22
409/04

TECHNICAL FIELDS SEARCHED (Int.Cl.)

C 07 D 333/22
409/04

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 14-03-1980 | CHOULY |

EPO Form 1503.1 06.78